# EUROPÄISCHE PATENTSCHRIFT

(11) **EP 1 911 407 B1**
(45) Veröffentlichungstag und Bekanntmachung des Hinweises auf die Patenterteilung: **16.12.2009**
(21) Anmeldenummer: 06021418.6
(22) Anmeldetag: 12.10.2006
(51) Int. Cl.: A61B 17/17, A61B 19/00, A61B 17/15

(54) **Modulares Patella-Instrumentarium**
Modular patellar resection instrument
Instrument modulaire pour la resection de la rotule

(43) Veröffentlichungstag der Anmeldung: 16.04.2008
(73) Patentinhaber: WALDEMAR LINK GmbH & Co. KG, 22339 Hamburg (DE)
(72) Erfinder: Keller, Arnold, 23863 Kayhude (DE)
(74) Vertreter: Glawe, Delfs, Moll

(56) Entgegenhaltungen:
- WO-A-96/25115
- US-A1- 5 129 907
- US-A1- 5 520 692

## Beschreibung

Die Erfindung betrifft ein modulares Instrumentarium zum Implantieren von Patella-Prothesen in verschiedenen Größen umfassend ein Halteinstrument, das zangenartig mit zwei Gliedern ausgebildet ist, wobei an dem vorderen Ende des einen Glieds eine Halteschale zum Zusammenwirken mit einer Vorderseite der Patella und an dem vorderen Ende des anderen Glieds eine Führung für eine Fräslehre vorgesehen ist, mehrere Fräslehren in verschiedenen Größen passend zu den Größen der Patella-Prothese, die auswechselbar über die Führung gehaltert sind, und Fräskörpern in verschiedenen Größen, wobei jeweils einer zu einer der Fräslehren gehört.

Erhält ein Patient eine Knieprothese, welche die Gelenkfunktion zwischen Femur und Tibia gewährleistet, so ist in der Regel auch die Implantation einer Patella-Prothese indiziert. Aufgrund der Anatomie des Kniegelenks wirkt die Rückseite des Patella-Knochens bei der Beugebewegung des Knies mit einem vorderen Bereich der Kondylen des Femurs zusammen. Sind die natürlichen Kondylen im Zuge der Implantation einer Knieprothese durch Kondylenkufen der Prothese ersetzt, so käme es zu einem Kontakt zwischen der natürlichen Patella und den Kondylenkufen der Prothese. Damit es nicht zu einem unerwünschten Kontakt zwischen natürlichem Knochenmaterial und dem künstlichen Prothesenmaterial, in der Regel Metall, kommt, wird an der Rückseite der Patella ein Implantat vorgesehen. Dieses fungiert als Gelenkfläche und wirkt dann mit der entsprechenden Gegenfläche an den Kondylenkufen der Knieprothese zusammen. Bedingt durch diese Funktion und durch die Form der natürlichen Patella weist die Patella-Prothese in der Regel eine knopfartige Gestalt auf. Ihre als Gelenkfläche fungierende Rückseite ist meist domartig konvex ausgeformt. Ihre zur Anlage an dem natürlichen Patella-Knochen dienende Vorderseite ist meist plan mit optionalen Verankerungsstiften. Die Implantation der Prothese erfolgt in der Weise, dass an der Rückseite des natürlichen Patella-Knochens Knochenmaterial durch Resektion abgetragen wird, wobei die Stärke des resezierten Knochenmaterials etwa der Dicke der Patella-Prothese entspricht. Damit wird erreicht, dass funktionelle Gelenkanatomie weitestgehend erhalten bleiben kann. Zur Resektion des Patella-Knochens kommen im Wesentlichen zwei Techniken zur Anwendung, nämlich Sägen oder Fräsen.

Ein Instrumentarium zum Sägen ist aus offenkundiger Vorbenutzung bekannt (Columbus Kniesystem von Aesculap Orthopedics). Hierbei wird die Dicke des Patella-Knochens bestimmt und der Patella-Knochen in eine Haltezange eingezwängt. Diese greift seitlich und an die Vorderseite des Patella-Knochens an. Dabei besteht die Gefahr, dass es zu Schädigungen von Gewebestrukturen, insbesondere Bändern, kommen kann, die ebenfalls seitlich mit der Patella verbunden sind. Die Haltezange beinhaltet eine Sägeführung. Damit kann der Operateur mittels einer oszillierenden Säge an der Rückseite der Patella eine Resektion von Knochenmaterial vornehmen. Die Stärke des zu resezierenden Knochenmaterials kann dabei mittels einer an dem Halteinstrument angeordneten Höheneinstellvorrichtung bestimmt werden. Die Resektion mittels einer oszillierenden Säge bringt es mit sich, dass beim Sägen entstehende Späne in die Umgebung gelangen und sich damit auch an anderem Gewebematerial anlagern können.

Eine vollständige Entfernung ist häufig nicht möglich. Ein dauernder Verbleib der Späne stellt ein Risiko für Infektionen oder andere Komplikationen dar. Weiter bietet die als Schlitz ausgestaltete Führung für die oszillierende Säge nur eine unzureichende Führung im Bereich der spanabtragenden Sägeblattspitze. Hält der Operateur das Instrument nicht genau rechtwinklig zu dem Schlitz der Sägeführung, so nimmt die oszillierende Säge entweder zu viel oder zu wenig Material von der Patella ab.

Weiter sind Instrumentarien zum Fräsen der Patella bekannt, beispielsweise aus US-B-6 866 667(Basis für den Oberbegriff des Anspruchs 1). Es umfasst ein zangenartiges Instrument mit einer Halteschale zum Unterstützen der Vorderseite der Patella an dem vorderen Ende eines Gliedes und einem Führungsring an dem vorderen Ende des anderen Gliedes, der zum rückseitigen Spannen der Patella gegen die Halteschale ausgebildet ist. Der Führungsring bestimmt eine Nullposition für eine Dickenmessung. Das Instrument weist weiter einen einstellbaren Stoppring auf, der je nach der Größe der zur Implantation vorgesehenen Patella-Prothese auf einen von vier verschiedenen Werten einstellbar ist. Je nach Einstellung ermöglicht dies ein unterschiedlich tiefes Eintauchen eines Fräskörpers durch den Führungsring, wodurch von der Patella-Rückseite Knochenmaterial entsprechend der eingestellten Dicke reseziert wird. Die Einstellung des Stopprings auf die zu resezierende Tiefe erfolgt durch den Operateur. Dabei kann es zu Einstellungsfehlern kommen. Insbesondere besteht die Gefahr, dass an dem Stoppring eine zu große Tiefe eingestellt wird, die zu einer größeren als der zur Implantation vorgesehenen Patella-Prothese gehört. Damit würde zu viel Knochenmaterial reseziert.

Der Erfindung liegt die Aufgabe zu Grunde ein Instrumentarium der eingangs genannten Art dahingehend zu verbessern, um die oben genannten Nachteilen vermeiden und insbesondere der Gefahr einer Einstellung einer zu großen Dicke bezogen auf die Größe der Patella-Prothese entgegenzuwirken.

Die erfindungsgemäße Lösung liegt in einem Instrumentarium mit den Merkmalen des unabhängigen Anspruchs. Vorteilhafte Weiterbildungen sind Gegenstand der abhängigen Ansprüche.

Bei einem modularen Instrumentarium zum Implantieren von Patella-Prothesen in verschiedenen Größen umfassend ein Halteinstrument, das zangenartig mit zwei Gliedern ausgebildet ist, wobei an dem vorderen Ende des einen Glieds eine Halteschale zum Zusammenwirken mit einer Vorderseite der Patella-Prothese und an dem vorderen Ende des anderen Glieds eine Aufnahme für ein Fräslehre vorgesehen ist, mehrere Fräslehren in verschiedenen Größen passend zu den Größen der Patella-Prothesen, die auswechselbar an der Aufnahme gehaltert sind, und mehrere Fräskörper in verschiedenen Größen, wobei jeweils einer davon zu einer der Fräslehren gehört, ist gemäß der Erfindung vorgesehen, dass Größenlehren mit einer umlaufenden Peilkante vorgesehen sind, deren Größe jeweils auf die einer der Fräskörper abgestimmt ist und die in die Fräslehren integriert sind, wobei mehrere Dickenringe vorgesehen sind, von denen jeweils einer auf dem in der Größe passenden Fräskörper aufsteckbar ist und deren Dicke derjenigen der jeweils dem Fräskörper zugeordneten Patella-Prothese entspricht.

Nachfolgend seien einige verwendete Begriffe erläutert.

Unter zangenartig wird ein Instrument verstanden, dessen zwei Glieder über ein in einem Mittenbereich der Glieder angeordnetes Gelenk miteinander verbunden sind. Meist wird es sich hierbei um ein Schwenkgelenk handeln, es kann aber auch ein Gelenk zur Parallelführung vorgesehen sein. Die Glieder weisen in dem hinteren Ende einen Griffteil auf, der zum kraftflüssigen Halten durch einen Operateur ausgebildet ist.

Unter Fräslehren werden Elemente verstanden, die einen Fräskörper in mindestens einer Richtung, vorzugsweise zwei Richtungen, führen, wobei die Fräskörper in einer dritten Richtung (die meist als Vorschubrichtung bezeichnet ist) beweglich sind.

Unter Patella-Prothesen in verschiedenen Größen wird verstanden, dass die Patella-Prothesen eine unterschiedliche Weite aufweisen. Die Patella-Prothesen bilden zwar kein Teil des erfindungsgemäßen Instrumentariums, jedoch ist ihre Weite bestimmend für die Weite des jeweils zugeordneten Fräskörpers. In der Regel ist es so, dass so viele Fräskörper mit verschiedenen Durchmessern in dem Instrumentarium vorgesehen sind, wie Patella-Prothesen in verschiedenen Größen mittels des Instrumentatriums implantiert werden sollen.

Unter Dickenringe werden ringartige Lehren verstanden, die eine bestimmte Höhe aufweisen. Die Höhe ist hierbei das Maß, das durch den Abstand der beiden Stirnseiten des Dickenrings bestimmt ist.

Der Erfindung liegt der Gedanke zu Grunde, eine Größenlehre in die Fräslehre zu integrieren, und mittels einer solchen kombinierten Fräs- und Größenlehre dem Operateur eine visuelle Bestimmung zu ermöglichen, ob die gewählte Fräslehre zu dem zu bearbeitenden Patella-Knochen passend ist. Die kombinierte Fräs- und Größenlehre vereint die Bestimmung der richtigen Prothesengröße und die Resektionsführung in einer einzigen Komponente. Der Operateur kann die größte in Frage kommende Prothesengröße auswählen und die entsprechende Fräslehre aus dem Instrumentarium an das Halteinstrument ansetzen. Mittels der in die Fräslehre integrierten Größenlehre kann der Operateur durch Vergleich mit der umlaufenden Peilkante prüfen, ob die Patella ausreichend groß für die gewählte Größe ist. Wenn die umlaufende Peilkante vollständig von der Patella überdeckt ist, dann kann die Patella diese Größe aufnehmen; fehlt eine Überdeckung an bestimmten Stellen, dann bedeutet dies, dass die Patella zu klein ist für die gewählte Größe. Im letzteren Fall ist dann die nächstkleinere Größe auszuwählen und mittels der nächstkleineren Fräslehre und der darin integrierten Größenlehre der Vorgang zu wiederholen. Ist schließlich mittels der in die Fräslehren integrierten Größenlehre die richtige Größe bestimmt, so ist auch der Durchmesser des zu verwendenden Fräskörpers festgelegt. Da zu jedem Fräskörper wiederum ein bestimmter Dickenring gehört, und nur dieser auf den jeweiligen Fräskörper passt, ist damit auch automatisch die Stärke des zu resezierenden Knochenmaterials an der Patella festgelegt.

Die Erfindung schafft eine lückenlose Kette beginnend von der Größenlehre zur Wahl der richtigen Prothesengröße bis hin zur Bestimmung der Tiefe, bis zu der der Fräskörper das Knochenmaterial an der Patella-Rückseite abträgt. Mit dem erfindungsgemäßen modularen Instrumentarium ist damit weder ein Messen noch ein Einstellen erforderlich. Fehler, die aus einer fehlerhaften Einstellung resultieren, können dank der Erfindung nicht mehr auftreten. Die Handhabung ist damit sowohl leichter wie auch sicherer.

Zweckmäßigerweise ist zusätzlich zu dem Dickenring ein verschieblicher Stoppring vorgesehen. Er dient dazu, einen Tiefenanschlag direkt an dem Fräskörper zu bilden. Dadurch braucht kein Tiefenanschlag an dem Halteinstrument vorgesehen zu sein. Die Anordnung des Stopprings direkt an dem Fräskörper hat weiter den Vorteil, dass Positionierungsungenauigkeiten des Halteinstruments sich nicht auf die Tiefenbestimmung auswirken. Um die Einstellung des Stopprings leicht handhabbar zu gestalten, ist zweckmäßigerweise eine Klemmeinrichtung an dem Stoppring vorgesehen. Sie ermöglicht ein einfaches Positionieren und Sichern des Stopprings in der gewünschten Position relativ zum Fräskörper.

Der Stoppring ist vorzugsweise kongruent zu dem Dickenring ausgebildet. Dies sichert eine große Berührungsfläche an den zur gegenseitigen Anlage kommenden Stirnseiten an Stopp- und Dickenring. Die kongruente Formgebung sorgt weiter für eine erleichterte Bedienbarkeit. Es ist intuitiv dem Operateur klar, dass der Dickenring so auf den Fräskörper (er passt erfindungsgemäß ja nur auf einen) aufgesteckt werden muss in der Weise, dass er an dem Stoppring anliegt. Der Gefahr von Fehlbedienungen wird dadurch weiter entgegengewirkt.

Zweckmäßigerweise weist das zangenartige Halteinstrument ein Gelenk auf, das eine Linearführung der Glieder bewirkt. Damit wird erreicht, dass die Halteschale des einen Glieds und die Fräslehre an dem anderen Glied sich linear entlang einer Achse aufeinander zu bewegen, und im Übrigen ihre Ausrichtung zueinander nicht ändern. Damit werden Winkelunterschiede, wie sie bei einer Führung entlang einer Kreisbogenform bei einem einfachen Schwenkgelenk auftreten würden, vermieden. Dies ermöglicht eine präzisere Führung und damit eine Schonung für die von dem erfindungsgemäßen Instrument beaufschlagten Bereiche der Patella und angrenzenden Bänder.

Zweckmäßigerweise sind Bohrlehreneinsätze in verschiedenen Größen vorgesehen. Sie ermöglichen es, geführte Bohrungen in die abgefräste Rückseite der Patella einzubringen. Derartige Bohrungen dienen zur Aufnahme von Verankerungsstiften der Patella-Prothese. Um eine bestimmte Winkellage dabei einzuhalten, sind zweckmäßigerweise die Bohrlehreneinsätze mit einer Winkelmarkierung versehen. Besonders zweckmäßig ist, wenn sie rastend ausgeführt ist, beispielsweise in Gestalt eines Stifts, der in richtiger Lage in eine passende Ausnehmung an der Fräslehre einrastet. Damit ist die Winkellage sicher festgelegt.

Vorzugsweise ist weiter eine an die Aufnahme ansetzbare Presseinrichtung vorgesehen. Sie dient dazu, nach dem Resezieren der Rückseite der Patella und gegebenenfalls Einbringen der Bohrungen die Patella-Prothese an dem vorgesehenen Implantationsort einzupressen. Die Anordnung der Presseinrichtung an dem erfindungsgemäßen Halteinstrument hat den Vorteil, dass letzteres zum Einpressen der Prothese nicht abgenommen zu werden braucht. Dies hat Vorteile, falls noch Nacharbeiten erforderlich sein sollten.

Vorzugsweise ist ein Bohraufsatz mit einem tiefenanschlagbegrenzten Bohrer vorgesehen. Dieser kann anstelle des Fräskörpers in die Fräslehre eingesetzt werden. Dies kann zweckmäßig sein, wenn bei einem sehr harten Patella-Knochen zuerst ein Brechen der harten Oberfläche erforderlich ist, bevor die eigentliche Fräsung mittels der Fräskörper durchgeführt wird. Der Tiefenanschlag an dem Bohrer sichert hierbei, dass der Bohrer nicht zu tief eindringt und damit die Planizität der für die Aufnahme der Patella-Prothese vorgesehenen Oberfläche beeinträchtigt. In der Regel genügt es, wenn der Tiefenanschlag so gewählt ist, dass ein ausreichender Abstand im Fall der kleinsten Größe der Patella-Prothese verbleibt. Gewünschtenfalls können aber auch Anschläge mit verschiedenen Tiefen oder verschiebliche Anschläge vorgesehen sein, um für die größeren Patella-Größen ein tieferes Freibrechen mittels des Bohrers zu ermöglichen.

Gemäß einer besonders zweckmäßigen Ausführungsform, die gegebenenfalls unabhängigen Schutz verdient, ist ein Sägelehrenmodul vorgesehen, das an die Aufnahme des Halteinstruments ansetzbar ist. Mit dem Sägelehrenmodul ist es ermöglicht, anstelle des Fräsens zum Resezieren des Patella-Knochens ein Sägen durchzuführen. Dies ist vor allem dann ein Vorteil, wenn sich intraoperativ herausstellt, dass die Resektion mittels eines Fräsers unzweckmäßig ist. Dann kann, ohne dass dazu das Instrumentarium gewechselt zu werden braucht, mittels des Sägelehrenmoduls die Resektion durchgeführt werden. Zweckmäßigerweise sind dazu Führungen an dem Größenlehrmodul vorgesehen. Sie sind so beschaffen, dass das Sägelehrenmodul in definierter Positionierung sicher an dem Größenlehrmodul halterbar ist. Vorzugsweise ist die Führung als eine Doppelführung ausgebildet, so dass das Sägelehrenmodul an zwei Seiten des Größenlehrmoduls angeordnet werden kann. Dies ermöglicht dem Operateur, je nach individuellen Zugangsverhältnissen eine optimale Positionierung des Sägelehrenmoduls zu wählen.

Vorzugsweise ist die Führung an dem Größenlehrmodul so ausgebildet, dass das Sägelehrenmodul höhenverschieblich an dem Größenlehrmodul geführt ist. Damit kann über eine Verschiebung des Sägelehrenmoduls relativ zu dem Größenlehrmodul die Stärke des abzutragenden Materials am Patella-Knochen bestimmt werden.

Um das Sägelehrenmodul in der richtigen Positionierung an dem Größenlehrmodul anzuordnen, ist zweckmäßigerweise eine Tiefenmesseinrichtung vorgesehen. Sie ist dazu ausgebildet, die Stärke des natürlichen Patella-Knochens zu ertasten und davon ausgehend die Höhe des Sägelehrenmoduls so zu bestimmen, dass beim Sägen Knochenmaterial in einer solchen Stärke abgetragen wird, wie es der Dicke der zu implantierenden Patella-Prothese entspricht. Die Tiefenmesseinrichtung weist dazu einen Taster auf, der von oben in die von der Größenlehre gebildete Öffnung hineinragt. Zweckmäßigerweise ist dieser Taster als ein Taststern mit einer Mehrzahl von strahlenartig angeordneten Tastkörpern ausgebildet. Die Tastkörper unterscheiden sich in ihren Abmessungen, wobei jeweils ein Tastkörper einer Größe der Patella-Prothesen zugeordnet ist. Die Erstreckung der Tastkörper in Radialrichtung entspricht dabei der Dicke der jeweiligen Patella-Prothese. Indem der Stern so gedreht wird, dass der Tastkörper für die zur Implantation vorgesehene Größe in Richtung zu der Patella-Halteschale zeigt, wird die Tiefenmesseinrichtung ohne weitere manuelle Einstellarbeiten automatisch richtig eingestellt. Es braucht dann nur noch die Tasteinrichtung mit dem Sägelehrenmodul so weit entlang der Führung verschoben zu werden, bist die Tasteinrichtung auf der Rückseite des Patella-Knochens aufliegt. Damit ist die Lage des Sägelehrenmoduls und damit die Ebene für die Knochenresektion mittels einer Säge eindeutig bestimmt.

Die Erfindung wird nachfolgend unter Bezugnahme auf die beigefügte Zeichnung erläutert, in der ein vorteilhaftes Ausführungsbeispiel der Erfindung dargestellt ist. Es zeigen:

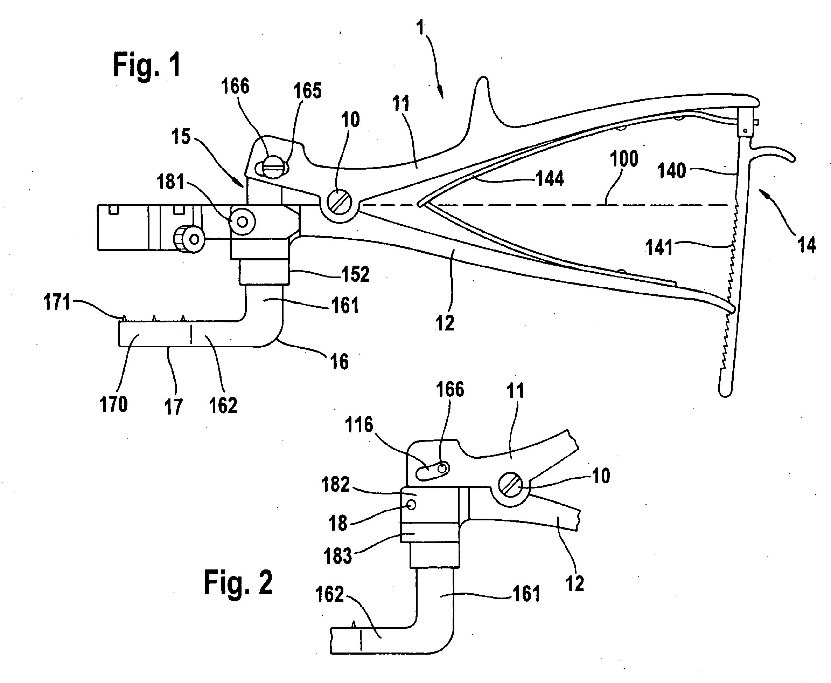

- Fig. 1:: eine Aufsicht auf ein Ausführungsbeispiel des erfindungsgemäßen Instruments;
- Fig. 2:: eine Detailansicht des Instruments aus Fig. 1;
- Fig. 3:: Größenlehren des Instrumentariums, zu dem das Instrument gemäß Fig. 1 gehört;
- Fig. 4:: Fräskörper des Instrumentariums in zwei verschiedenen Größen;
- Fig. 5:: Dickenringe in verschiedenen Stärken und einen Fräskörper;
- Fig. 6:: Bohrlehren des erfindungsgemäßen Instrumentariums;
- Fig. 7:: eine Patella-Prothese zur Implantation durch das Instrumentarium;
- Fig. 8:: Bestimmen einer richtigen Prothesengröße mit dem erfindungsgemäßen Instrumentarium;
- Fig. 9:: Einsetzen eines Fräskörpers zur Bestimmung einer Einfangsposition;
- Fig. 10:: Fräsen der Patella;

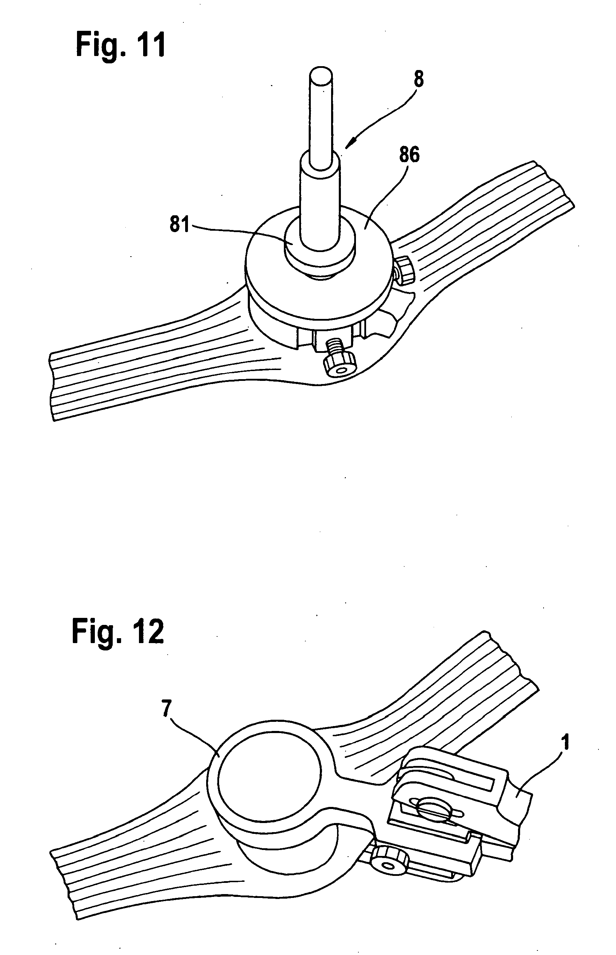

- Fig. 11:: Brechen einer Kuppenspitze der Patella;
- Fig. 12:: Einpressen der Patella-Prothese;
- Fig. 13:: eine perspektivische Ansicht einer Sägelehre für das erfindungsgemäße Instrumentarium; und
- Fig. 14:: das an die Patella angesetzte Instrumentarium mit der Sägelehre.

Ein erfindungsgemäßes Instrumentarium umfasst ein Halteinstrument 1, eine Mehrzahl von Fräslehren 3 mit jeweils einer integrierten Größenlehre 2, eine entsprechende Anzahl von größenmäßig auf die Fräs- und Größenlehren 2, 3 abgestimmten Fräskörper 4, jeweils einem der Fräskörper 4 zugeordneten Dickeringe 5, eine entsprechende Anzahl von Bohrlehren 6 sowie Einpresseinsätzen 7, einen Bohrer 8, sowie ein Sägenlehrenmodul 9.

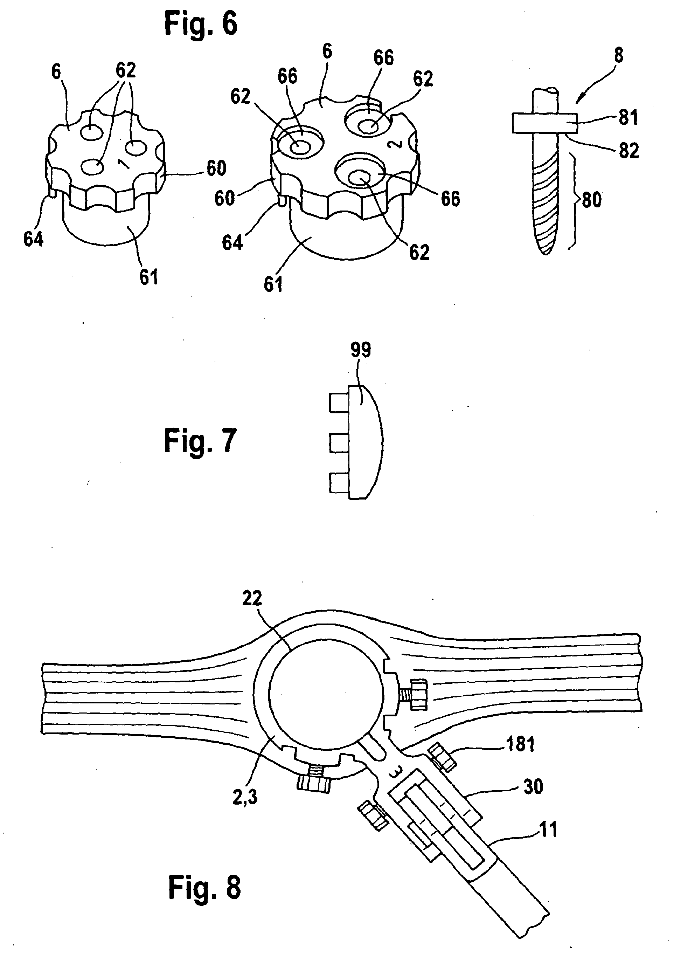

Zur Implantation sind Patella-Prothesen in verschiedenen Größen vorgesehen. Ein Beispiel ist in Fig. 7 dargestellt. Die Patella-Prothese 99 umfasst einen Tragkörper mit einer balligen Vorderseite und mehrere Zapfen an der Rückseite.

Das Halteinstrument 1 ist zangenartig mit zwei beweglich über ein Schwenkgelenk 10 angeordneten Gliedern 11, 12 ausgeführt. Beide Glieder sind in ihrem hinteren Teil griffartig ausgebildet, und am hinteren Ende ist eine Arretiereinrichtung 14 zum Fixieren des Halteinstruments 1 in einer gespannten Position angeordnet. Der Arretiereinrichtung 14 weist eine schwenkbar an dem Glied 11 angeordnete Stange 140 mit einer Sägezahnverzahnung 141 an ihrer Vorderseite auf. Diese fasst in eine entsprechende Kante (nicht dargestellt) einer Ausnehmung an der hinteren Spitze des Glieds 12. Zum Aufbringen einer die Glieder 11, 12 auseinander treibenden Kraft ist eine V-förmig geformte Blattfeder 144 vorgesehen.

Das vordere Ende der Glieder 11, 12 ist als eine Linearführung 15 ausgebildet. Sie umfasst eine Führungshülse 152 in dem vorderen Ende des Glieds 12, in der ein Schenkel 161 eines L-förmig geformten Haltewinkels 16 längsverschieblich in einer Querachse des Instruments 1 geführt ist. Unter Querachse wird hierbei eine Achse verstanden, die senkrecht auf der Längsachse 100 des Halteinstruments 1 steht, welche sich von vorne nach hinten durch das Schwenkgelenk 10 erstreckt. Der andere Schenkel 162 des Haltewinkels 16 erstreckt sich parallel zur Längsachse 100 nach vorne und er weist an seinem vorderen Ende eine Halteschale 17 auf. Sie weist einen ringförmigen Rand 170 mit mehreren gleichmäßig darauf verteilten, zur Hauptachse 100 des Instruments weisenden Dornen 171 auf. Die Halteschale 17 ist dazu ausgebildet, beim Ansetzen des Halteinstruments 1 die Rückseite der Patella-Sehne zu erfassen und zu fixieren. Das von der Halteschale 17 entfernte Ende des Schenkels 161 ist über ein Schwenklager 165 mit dem vorderen Ende des Glieds 11 verbunden. Das Schwenklager 165 kann in seiner einfachsten Form als ein gekrümmter Schlitz 116 an dem vorderen Ende des Glieds 11 ausgeführt sein, indem der Schaft an einer Schraube 166 geführt ist. Krümmung und Orientierung des Schlitzes 116 sind so gewählt, dass außer der Schwenkbeweglichkeit auch ein Längenausgleich erreicht wird. An dem vorderen Ende des Glieds 12 ist außer der Führungshülse 152 eine Universalaufnahme 18 ausgebildet. Sie dient dazu, die Größen- und Fräslehren 2, 3, das Pressmodul 7 und das Sägelehrmodul 8 positionsgenau an dem Instrument 1 zu haltern. Die Universalhalterung 18 ist gestuft ausgeführt und weist einen oberen, dem Glied 11 zugewandten Abschnitt auf, indem die Materialstärke in Richtung zur Mittelachse 100 gesehen verringert ist. Dieser Abschnitt wird als verdünnter Abschnitt 182 bezeichnet, während ein sich daran in Richtung des Glieds 12 anschließender Abschnitt als verdickter Abschnitt 183 bezeichnet wird.

Zur Sicherung gegenüber einer unbeabsichtigten Lockerung oder Verschiebung ist eine Klemmschraube 181 an dem jeweiligen Einsatz bzw. Modul vorgesehen.

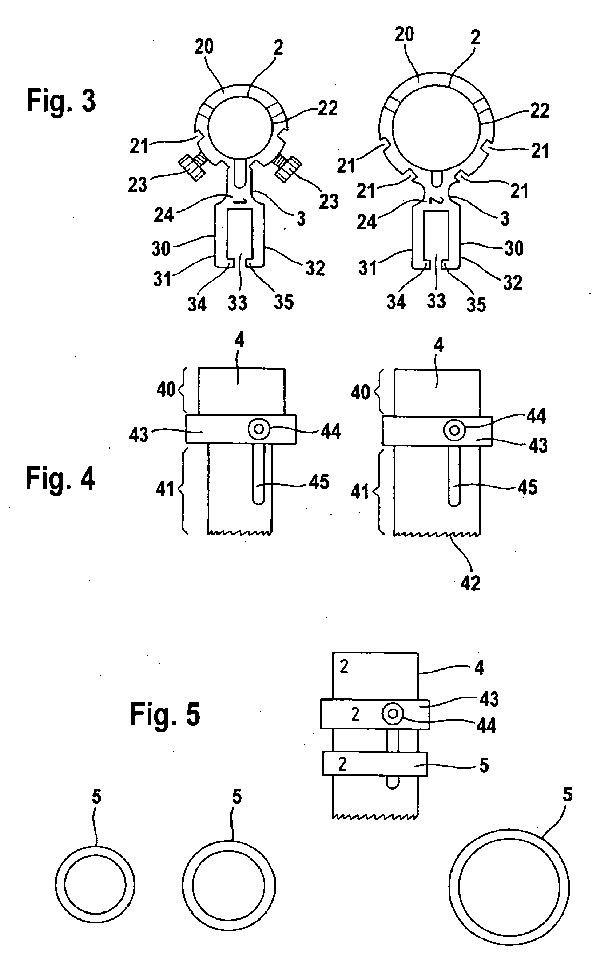

Die Größen- und Fräslehren 2, 3 sind in verschiedenen Größen vorgesehen, von denen zwei aus einem Beispielsatz von insgesamt vier in Fig. 3 dargestellt sind. Sie bestehen im Wesentlichen aus einer Ringhülse 20 und einer sich davon radial nach außen erstreckenden Halteklammer 30. Letztere weist zwei Halteschenkel 31, 32 auf, die an den U-förmigen Raum 33 zur passgenauen Aufnahme der Universalhalterung 18 umgrenzen. Weiter sind an den äußeren Enden der Schenkel 31, 32 einander zugewandte Vorsprünge 34, 35 angeordnet, welche die Universalhalterung 18 im montierten Zustand umfassen. An mindestens einem der Schenkel ist eine Aufnahme für die Arretierschraube 181 angeordnet. Die Weite zwischen den Vorsprüngen 34, 35 ist so groß bemessen, dass sie für einen Durchgang der Universalhalterung 18 in ihrem verdünnten Abschnitt ausreicht, aber den Durchgang in dem verdickten Abschnitt verhindert. Die Ringhülse 20 weist an ihrem Mantel zwei Paare von schrägwinklig gegenüberliegend angeordneten Führungsnuten 21 auf, die sich in axialer Richtung über die gesamte Höhe der Ringhülse 20 erstrecken. Zwischen den beiden Führungsnuten 21 eines Paares ist jeweils eine Klemmschraube 23 in einem Gewinde angeordnet. Diese Einrichtung dient zur Befestigung zusätzlicher Module, wie später noch erläutert werden wird. An der Oberseite eines Übergangsbereichs zwischen der Ringhülse 20 und dem Halteschenkel 30 ist eine unverwechselbare Größenmarkierung 24 (wie zum Beispiel die Ziffern 1, 2, 3 oder 4) angeordnet, welche die jeweilige Größe der Größen- und Fräslehre 2, 3 eindeutig kennzeichnen. Eine besondere Bedeutung kommt der umlaufenden inneren Unterkante 22 der Ringhülse 20 zu. Sie ist als eine Peilkante ausgeführt. Das bedeutet, dass ihre Kontur genau passend zu der Außenkontur der Patella-Prothese 99 ausgeführt ist. Dies bedeutet, das die Peilkante 22 genau den Bereich umgrenzt, auf dem die Patella-Prothese 99 zu implantieren ist. Zu jeder Größe der Patella-Prothesen 99 gibt es eine Größen- und Fräslehre 2, 3 deren Peilkante 22 genau der Außenkontur der jeweiligen Patella-Prothesen 99 folgt. Damit ist eine eindeutige Zuordnung zwischen der jeweiligen Größen- und Fräslehre 2, 3 und einer Größe der Patella-Prothesen 99 geschaffen. Dies ermöglicht dem Operateur eine einfache und sichere Auswahl der richtigen Größe der Patella-Prothese 99 und gleichzeitig verwechselungssichere Festlegung der zur Implantation dieser Größen zu verwendenden Elemente des erfindungsgemäßen Instrumentatriums. Dazu hält der Operateur, zweckmäßigerweise beginnend mit der größten Größe, nacheinander die Größenlehren 2 über den Patella-Knochen, auf dem die Patella-Prothese 99 zu implantieren ist. Es braucht dann nur geprüft zu werden, ob die Peilkante 22 vollständig von dem Patella-Knochen bedeckt ist oder nicht. Ist dies nicht der Fall, dann ist der Patella-Knochen zu klein für die geprüfte Größe, und es ist die nähst kleinere Größe zu wählen, womit mit der Vorgang wiederholt wird, bis schließlich diejenige Größenlehre 2 gefunden ist, bei der die Peilkante 22 gerade noch vollständig von dem Patella-Knochen überdeckt ist. Damit ist die richtige Größe der Patella-Prothese gefunden. Diese Größenlehre 2 kann nun an dem Halteinstrument 1 mittels der Universalhalterung 18 befestigt werden. Da die Größenlehre 2 erfindungsgemäß gleichzeitig auch Fräslehre 3 ist, zu der nur genau ein Fräskörper 4 passt, ist damit gleichzeitig sichergestellt, dass die richtige Fräslehre und damit auch der richtige Fräskörper 4 verwendet wird. Fehler, bei deren aufgrund von Verwechslungen ein zu großer Fräskörper 4 verwendet wird und damit unwiederbringlich Knochenmaterial fälschlich abgetragen wird, sind damit ausgeschlossen.

Ist die somit eindeutig bezüglich ihrer Größe bestimmte Größen- und Fräslehre 2, 3 an dem Halteinstrument 1 fixiert, kann die Rückseite des Patella-Knochens zur Implantation der Patella-Prothese 99 in der ausgewählten Größe präpariert werden. Bei harten und sehr knöchernen Rückseitenoberflächen des Patella-Knochens kann optional vorgesehen sein, mittels des Bohrers 8 die Kuppenspitze der Patella-Rückseite zu brechen, und damit den Zugang für die Fräskörper 4 zu vereinfachen. Der Bohrer 8 weist an seinem vorderen Abschnitt einen Spiralbereich 80 auf, der in üblicherweise wie für einen Bohrer ausgeführt ist. An diesen schließt sich nach hinten ein Bund 81 an. Seine dem Spiralabschnitt 80 zugewandte Stirnseite 82 fungiert als Anschlagfläche auf einer Oberseite einer Anschlagplatte 86. Die Anschlagplatte 86 ist als kreisrunde Scheibe ausgeführt und liegt deckelartig auf der Ringhülse 20 der gewählten Größe- und Fräslehre 2, 3 auf. Sie weist eine Zentralöffnung auf, deren weiterführenden Durchgang des Spiralabschnitts 80 des Bohrers bemessen ist, aber kleiner ist als der Durchmesser des Bunds 81. Damit kann beim Bohren der Bohrer 8 lediglich so tief in die Ringhülse 20 eintauchen, bis der Bund 81 mit seiner Stirnseite 82 auf der Platte 86 anschlägt. Die Höhe der Ringhülse 20 ist dabei je nach Größe der jeweiligen Größen- und Fräslehre 2,3 so gewählt, dass der Bohrer 8 bei Erreichen des Anschlags lediglich den oberen Spitzenbereich des Patella-Knochens entfernt. Damit ist sichergestellt, dass der Bohrer 8 nicht über die eigentliche Implantationstiefe hinaus Knochenmaterial abträgt.

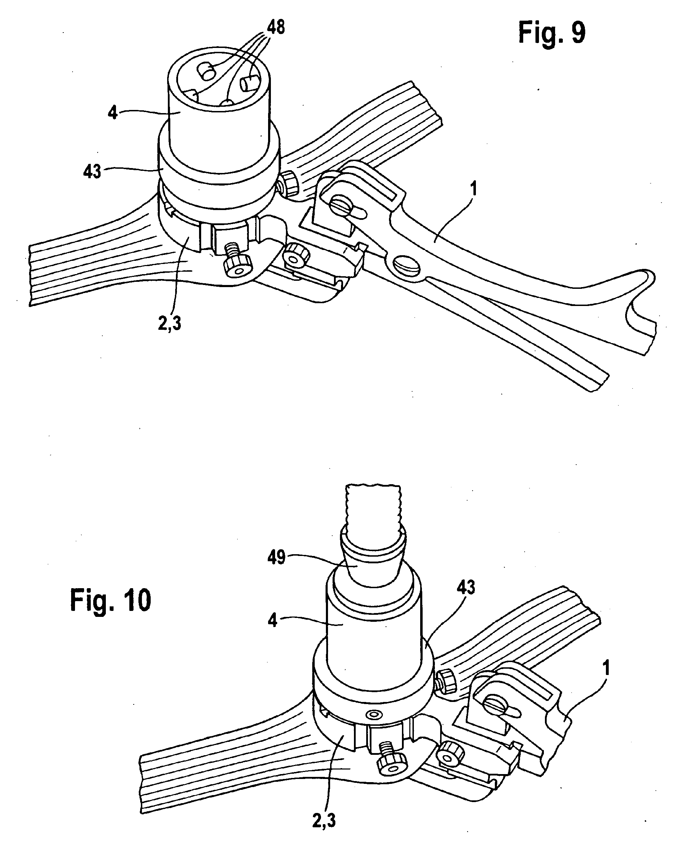

Die in dem dargestellten Ausführungsbeispiel vier verschieden großen Fräskörper 4 weisen jeweils einen Ober- und einen Unterabschnitt 40, 41 auf. Der Oberabschnitt 40 ist hülsenartig ausgeführt und weist an seiner Innenseite 4 radial nach innen weisende Vorsprünge 48 (siehe Fig. 9) als Kupplungselemente für ein Antriebswerkzeug 48 (siehe Fig. 10) auf. Über sie wird der Fräskörper 4 angetrieben. Der obere Abschnitt 40 weist bei allen Fräskörpern 4 dieselbe Weite auf. Der untere Abschnitt 41 ist zylinderartig geformt mit einer Fräsverzahnung 42 an seiner unteren Stirnfläche. Der Durchmesser des unteren Abschnitts 41 ist je nach der Größe des jeweiligen Fräskörpers 4 verschieden. Er entspricht jeweils dem Durchmesser der verschiedenen Größen der Patella-Prothese 99. Dieser Durchmesser entspricht bis auf ein geringes Spiel zur Vermeidung von Klemmung dem Innendurchmesser der Ringhülse 20 der jeweils zugeordneten Größen- und Fräslehre 2, 3. Auf dem unteren Abschnitt 41 ist ein Stoppring 43 aufgeschoben. Er ist über eine Klemmschraube längsverschieblich in einer Axialnut 45 am Mantel des unteren Bereichs 41 geführt. Der Stoppring kann in eine beliebige Position entlang der Nut 45 geschoben werden, und dort mittels der Klemmschraube 44 fixiert werden. Der Innendurchmesser des Stopprings 43 ist so gewählt, dass er praktisch spielfrei auf dem unteren Abschnitt 41 sitzt; sein Außendurchmesser ist so gewählt, dass er mit demjenigen der Ringhülse 20 der zugeordneten Größen- und Fräslehre 2, 3 übereinstimmt. Zum Fräsen wird der Fräskörper 4 in die zuvor gewählte Größen- und Fräslehre 2, 3 eingesetzt, bis er mit seiner Stirnseite mit der Fräsverzahnung 42 auf der Spitze der Rückseite des Patella-Knochens aufliegt. Von dieser Position aus ist die Abtragungstiefe zu bestimmen, welche gleich der Dicke des Tragelements der Patella-Prothese 99 ist. Mittels des Fräskörpers 4 abzutragen ist Material einer solchen Stärke, welche der Dicke des Tragkörpers der Patella-Prothese 99 entspricht.

Es ist für jede Größe der Patella-Prothese 99 jeweils ein Dickenring 5 vorgesehen. Seine Höhe entspricht jeweils der Stärke des Tragkörpers der Patella-Prothese 99, weshalb sie auch als "Dicke" bezeichnet wird. Der Innendurchmesser der Innenringe 5 ist jeweils so gewählt, dass er mit dem Außendurchmesser des unteren Bereichs 41 desjenigen Fräskörpers 4 übereinstimmt, der zu der jeweiligen Größe der Patella-Prothese 99 gehört. Damit ist eine eindeutige und verwechslungssichere Zuordnung zwischen Dickenringen 5 und Fräskörpern 4 in Bezug auf die jeweilige Größe der Patella-Prothese 99 gewährleistet. Es ist vorgesehen, dass dem ausgewählten Fräskörper 4 zugeordnete Dickenring 5 von unten auf den unteren Abschnitt 41 aufgeschoben und zur Anlage an den Stoppring 43 gebracht wird. Der ausgewählte Fräskörper 4 mit dem aufgeschobenen Dickenring 5 wird dann in die zugeordnete Größen- und Fräslehre 2, 3 eingesetzt und soweit vorgeschoben, dass die Stirnseite des unteren Abschnitts 41 mit den Fräsverzahnung 42 auf der Spitze der Rückseite des Patella-Knochens aufliegt. Der Stoppring 43 wird dann soweit nach unten zu der Ringhülse 20 hinbewegt, bis der Dickenring 5 an der Oberseite der Ringhülse 20 anschlägt. In dieser Position wird die Klemmschraube 44 des Stopprings 43 festgezogen. Damit ist ausgehend von dem durch die Höhe der Spitze des Patella-Knochens bestimmten Nullpunkt und der durch den Dickenring 5 repräsentierten Dicke der zu implantierenden Patella-Prothese die Tiefe des mit dem Fräskörper 4 auszuführenden Fräsvorgangs bestimmt. Der Stoppring 43 fungiert damit nun als Tiefenanschlag. Der Fräskörper 4 wird aus der Fräs- und Größenlehre entnommen, um den Dickenring zu entfernen. Er wird dann wieder eingesetzt, und kann mit dem Antriebswerkzeug verbunden werden. Das Fräsen kann dann soweit durchgeführt werden, bis der Fräskörper 4 soweit vorgeschoben ist, dass der Stoppring 43 an der Oberseite der Ringhülse 20 aufliegt (siehe Figur 9). Damit ist die maximale Frästiefe entsprechend der Dicke der Patella-Prothese 99 in der gewählten Größe erreicht. Fehler, die durch ein versehentliches zu tiefes Fräsen sich ergeben könnten, werden auf diese Weise sicher ausgeschlossen.

Der Fräskörper 4 kann nun entfernt werden, und Befestigungsbohrungen mittels der Bohrlehren 6 angebracht werden. Die Bohrlehren 6 sind von etwa pilzförmiger Gestalt mit einem unteren Abschnitt 61, dessen Durchmesser auf den Innendurchmesser der Ringhülse 20 der jeweiligen Größen- und Fräslehre 2, 3 abgestimmt ist. Ein oberer Abschnitt 60 steht bundartig hervor und weist einen Durchmesser auf, der dem Außendurchmesser der Ringhülse 20 entspricht. An der Oberseite der Bohrlehre 6 sind drei um 120° versetzte Durchgangsbohrungen 62 angeordnet, die sich durch den oberen Abschnitt 60 unter dem unteren Abschnitt 61 hindurch erstrecken. Je nach Größe der Bohrlehre kann eine den Rand der jeweiligen Bohrung 62 umschließende Ansenkung 66 vorgesehen sein. Sie ist dazu ausgebildet, dass sie als Tiefenanschlag für einen durch die Bohrungen 62 gesteckten Bohrer fungieren. Damit wird erreicht, dass der Bohrer bezogen auf die als Referenzfläche fungierende Unterseite des oberen Abschnitts 60 eine vorbestimmbare Tiefe erreichen kann. Ein zu tiefes Bohren der Befestigungslöcher für die Zapfen der Patella-Prothese 99 kann damit auf einfache und sichere Weise verhindert werden. An der Referenzfläche ist ein Stift 64 angeordnet, der in eine entsprechende Ausnehmung an der Größen-/Fräslehre 2, 3 eingreift und als Winkelmarkierung fungiert.

Mit dem Entfernen der Bohrlehre 6 ist der Patella-Knochen für die Implantation der Patella-Prothese 99 der gewählten Größe präpariert. Die Stärke des abgetragenen Knochenmaterials entspricht genau der Dicke des Tragelements der Patella-Prothese 99. Die Tiefe der Löcher entspricht der Länge der Befestigungszapfen 96 der Patella-Prothese 99. Die mit herkömmlichen Instrumentarien verbundene Gefahr von zu tief ausgeführten Fräsungen oder Bohrungen besteht nicht. Dank des erfindungsgemäßen Instrumentariums wird vielmehr erreicht, dass die Stärke des abgetragenen Knochenmaterials genau derjenigen zu implantierenden Größe der Patella-Prothese 99 entspricht, so dass eine sichere und anatomisch richtige Positionierung der Patella-Prothese 99 erreicht wird.

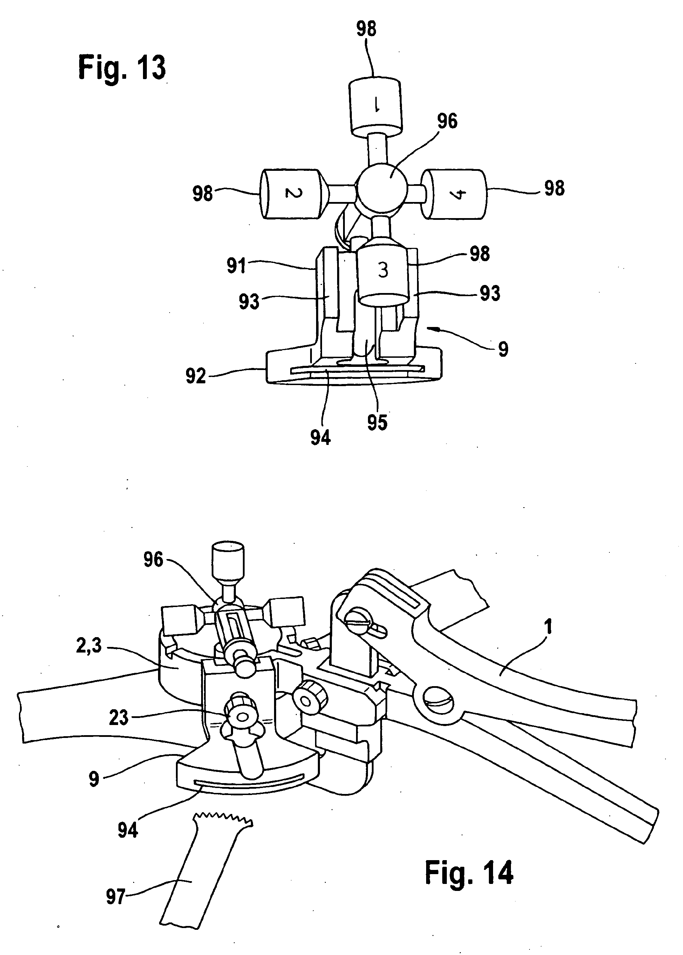

In Fig. 13, 14 ist das Sägelehrenmodul 9 dargestellt, welches zur Durchführung der alternativen Resektionsmethode des Sägens anstelle des Fräsens vorgesehen ist. Das Sägelehrenmodul 9 weist einen L-förmigen Grundkörper mit einem Basisschenkel 91 und einem Führungsschenkel 92 auf. Der Basisschenkel 91 weist an seiner dem Führungsschenkel 92 gegenüberliegenden Seite Führungsleisten 93 auf, die zum formschlüssigen Eingreifen in die Führungsnuten 21 der kombinierten Fräs-/Größenlehre 2, 3 ausgebildet sind. Sie ermöglichen eine höhenverstellbare Anordnung des Sägenlehrenmoduls 9 an der Fräs-/Größenlehre 2, 3. Zwischen den Führungsleisten 93 ist ein parallel laufender Schlitz 95 angeordnet. Durch diesen kann die Klemmschraube 23 gesteckt werden, um so durch Festziehen das Sägelehrenmodul 9 in seiner Position zu arretieren. Der Führungsschenkel 92 weist einen Führungsschlitz 94 auf, der eine Schneidebene für ein Sägeblatt 97 bestimmt. Die Schneidebene ist hierbei so orientiert, dass ihr Normalenvektor in Richtung der Führungsleisten 93 verläuft, so dass bei montiertem Sägenlehrenmodul die Schneidebene parallel zur der Oberkante des Halterings 170 liegt.

An dem Basisschenkel 91 ist an dem dem Führungsschenkel 92 gegenüberliegenden Ende ein Taststern 96 angeordnet. Er weist vier um 90° versetzte, in Radialrichtung weisende Tastkörper 98 auf. Sie weisen eine unterschiedliche Länge auf, wobei jeweils einer der Tastkörper 98 einer Größe der Prothese 99 zugeordnet ist. Die unterschiedlichen Längen bilden dabei die unterschiedlichen Dicken der Prothesen 99 in den verschiedenen Größen ab. Der Taststern 96 ist drehbar so angeordnet, dass jeweils einer der Tastkörper 98 in eine sich in Richtung zu dem Führungsschlitz 94 hinweisende Stellung bringbar ist. Dieser Tastkörper ist der aktive Tastkörper. Seine Bezeichnung stimmt mit derjenigen des verwendeten Größen-/Fräslehrmoduls 2, 3 überein.

Bei der Operation braucht der Chirurg lediglich das Sägelehrmodul 9 soweit entlang seiner von den in den Führungsnuten 21 laufenden Führungsleisten 93 gebildeten Führung nach unten zu verschieben, bis der zu der verwendeten Größen-/Fräslehre 2, 3 zugehörende Tastkörper 98 mit seiner Außenfläche oben auf der Rückseite des zu resezierenden Patella-Knochens aufsitzt. Damit ist die korrekte Position des Sägelehrmoduls 9 gefunden. Die Klemmschraube 23 kann fixiert werden. Der Führungsschlitz 94 für das Sägeblatt 97 befindet sich dann in der richtigen Höhe, nämlich ein um die Stärke der Patella-Prothese 99 entsprechendes Maß tiefer als der höchste Punkt des Patella-Knochens. Die Resektion durch Sägen kann jetzt auf einfache Weise durch Einführen des Sägeblatts 97 in den Führungsschlitz 94 und Durchführen der Sägung erfolgen. Das Instrument mit dem erfindungsgemäßen Sägelehrmodul 9 braucht dabei nicht abgesetzt zu werden, sondern das Sägen kann direkt in einem Zug durchgeführt werden.

Die Führungsnuten 21 sind so an dem Größen-/Fräslehrmodul 2, 3 angeordnet, dass das Sägelehrmodul 9 beidseitig in einem 45°-Winkel nach hinten bezogen auf die Längsachse 100 des Instruments wegsteht. Diese Anordnung ergibt den bestmöglichen Kompromiss aus Zugänglichkeit und günstiger Sägeblattführung. Mit dem erfindungsgemäßen Sägelehrmodul 9 kann damit das Sägen einfach und genau durchgeführt werden, wobei Fehlermöglichkeiten auf ein Minimum beschränkt sind.

## Patentansprüche

1. Modulares Instrumentarium zum Implantieren von Patella-Prothesen (99) in verschiedenen Größen umfassend ein Halteinstrument (1), das zangenartig mit zwei Gliedern (11, 12) ausgebildet ist, wobei an dem vorderen Ende des einen Glieds (11) eine Halteschale (17) zum Zusammenwirken mit einer Vorderseite der Patella-Prothese (99) und an dem vorderen Ende des anderen Glieds (12) eine Aufnahme für eine Fräslehre (3) vorgesehen ist, mehrere Fräslehren (3) in verschiedenen Größen passend zu den Größen der Patella-Prothesen (99), die auswechselbar an der Aufnahme (18) gehaltert sind, und mehrere Fräskörper (4) in verschiedenen Größen, wobei jeweils einer davon zu einer der Fräslehren (3) gehört,
**dadurch gekennzeichnet, dass**
Größenlehren (2) mit einer umlaufenden Peilkante (22) vorgesehen sind, deren Größe jeweils auf einen der Fräskörper (4) abgestimmt ist und die in die Fräslehren (3) integriert sind, wobei mehrere Dickenringe (5) vorgesehen sind, von denen jeweils einer auf dem in der Größe passenden Fräskörper (4) aufsteckbar ist und deren Dicke derjenigen der jeweils dem Fräskörper zugeordneten Patella-Prothese (99) entspricht.

2. Modulares Instrumentarium nach Anspruch 1,
**dadurch gekennzeichnet, dass**
an den Fräskörpern (4) zusätzlich zu dem Dickenring (5) ein verschieblicher Stoppring (43) vorgesehen ist.

3. Modulares Instrumentarium nach Anspruch 2,
**dadurch gekennzeichnet, dass**
eine Klemmeinrichtung (44) an dem Stoppring (43) vorgesehen ist.

4. Modulares Instrumentarium nach Anspruch 2 oder 3,
**dadurch gekennzeichnet, dass**
der Stoppring (43) kongruent zu dem Dickenring (5) ausgebildet ist.

5. Modulares Instrumentarium nach einem der vorangehenden Ansprüche,
**dadurch gekennzeichnet, dass**
das Instrument (1) ein Gelenk (10) aufweist, das zu einer Linearführung der Glieder (11, 12) ausgebildet ist.

6. Modulares Instrumentarium nach einem der vorangehenden Ansprüche,
**dadurch gekennzeichnet, dass**
Bohrlehreneinsätze (6) in verschiedenen Größen vorgesehen sind.

7. Modulares Instrumentarium nach Anspruch 6,
**dadurch gekennzeichnet, dass**
die Bohrlehreneinsätze (6) mit einer Winkelmarkierung (64) versehen sind.

8. Modulares Instrumentarium nach einem der vorangehenden Ansprüche,
**dadurch gekennzeichnet, dass**
eine an die Aufnahme (18) ansetzbare Presseinrichtung (7) vorgesehen ist.

9. Modulares Instrumentarium nach einem der vorangehenden Ansprüche,
**dadurch gekennzeichnet, dass**
ein Bohraufsatz (86) mit einem tiefenanschlagbegrenzten Bohrer (8) vorgesehen ist.

10. Modulares Instrumentarium nach einem der vorangehenden Ansprüche,
**dadurch gekennzeichnet, dass**
ein Sägelehrmodul (9) vorgesehen ist, das an die Aufnahme (18) des Halteinstruments (1) ansetzbar ist.

11. Modulares Instrumentarium nach Anspruch 10,
**dadurch gekennzeichnet, dass**
Führungen für das Sägelehrmodul (9) an dem Größenlehrmodul (2) vorgesehen sind.

12. Modulares Instrumentarium nach Anspruch 10 oder 11,
**dadurch gekennzeichnet, dass**
eine Tiefenmesseinrichtung an dem Sägelehrmodul (9) vorgesehen ist.

13. Modulares Instrumentarium nach Anspruch 12,
**dadurch gekennzeichnet, dass**
die Tiefenmesseinrichtung einen Taster aufweist, der von oben in eine von der Größenlehre (2) gebildete Füllung hineinragt.

14. Modulares Instrumentarium nach Anspruch 13,
**dadurch gekennzeichnet, dass**
der Taster als ein Taststern (96) mit einer Mehrzahl von strahlenartig angeordneten Tastkörpern (98) ausgebildet ist, die jeweils eine unterschiedliche Größe aufweisen.

## Claims

1. Modular instrument set for implanting patella prostheses (99) of various sizes, comprising a retaining instrument (1) which is designed like forceps with two members (11, 12), the front end of one member (11) being provided with a retention plate (17) for engagement with a front face of the patella prosthesis (99), and the front end of the other member (12) being provided with a holder for a milling gauge (3), and comprising several milling gauges (3) which are available in various sizes matching the sizes of the patella prostheses (99) and are mounted exchangeably on the holder (18), and several milling bodies (4) of various sizes, one each of which belongs to one of the milling gauges (3), **characterized in that** size gauges (2) with a circumferential marker edge (22) are provided whose sizes are in each case adapted to one of the milling bodies (4) and which are integrated into the milling gauges (3), and several thickness rings (5) are provided which can each be placed on the milling body (4) of matching size and whose thickness corresponds to that of the patella prosthesis (99) associated with the milling body.

2. Modular instrument set according to Claim 1, **characterized in that** a displaceable stop ring (43) is provided on the milling bodies (4) in addition to the thickness ring (5).

3. Modular instrument set according to Claim 2, **characterized in that** a clamping means (44) is provided on the stop ring (43).

4. Modular instrument set according to Claim 2 or 3, **characterized in that** the stop ring (43) is designed congruent to the thickness ring (5).

5. Modular instrument set according to one of the preceding claims, **characterized in that** the instrument (1) has a joint (10) which is designed for linear guiding of the members (11, 12).

6. Modular instrument set according to one of the preceding claims, **characterized in that** drill jigs (6) are provided in various sizes.

7. Modular instrument set according to Claim 6, **characterized in that** the drill jigs (6) are provided with an angle marking (64).

8. Modular instrument set according to one of the preceding claims, **characterized in that** a pressing device (7) is provided that can be mounted on the holder (18).

9. Modular instrument set according to one of the preceding claims, **characterized in that** a drill fixture (86) is provided which has a drill (8) limited by a depth stop.

10. Modular instrument set according to one of the preceding claims, **characterized in that** a saw jig module (9) is provided which can be mounted on the holder (18) of the retaining instrument (1).

11. Modular instrument set according to Claim 10, **characterized in that** guides for the saw jig module (9) are provided on the size gauge module (2).

12. Modular instrument set according to Claim 10 or 11, **characterized in that** a depth-measuring device is provided on the saw jig module (9).

13. Modular instrument set according to Claim 12, **characterized in that** the depth-measuring device has a probe that protrudes downward into an opening formed by the size gauge (2).

14. Modular instrument set according to Claim 13, **characterized in that** the probe is in the form of a probe star (96) having a plurality of probe bodies (98) that are arranged in a radiating pattern and are each of a different size.

## Revendications

1. Système d'instrument modulaire pour l'implantation de prothèses de rotule (99) de différentes tailles, comprenant un instrument de retenue (1) qui est réalisé sous forme de pince avec deux organes (11, 12), une coque de retenue (17) étant prévue à l'extrémité avant de l'un des organes (11), pour coopérer avec un côté avant de la prothèse de rotule (99) et un logement pour un gabarit de fraisage (3) étant prévu à l'extrémité avant de l'autre organe (12), plusieurs gabarits de fraisage (3) de différentes tailles adaptés aux tailles des prothèses de rotule (99), qui sont retenues de manière remplaçable sur le logement (18), et plusieurs corps de fraisage (4) de différentes tailles, à chaque fois l'un d'entre eux appartenant à l'un des gabarits de fraisage (3), **caractérisé en ce que** des gabarits de taille (2) avec une arête de sondage périphérique (22) étant prévus, leur taille étant à chaque fois adaptée à l'un des corps de fraisage (4) et ces gabarits de taille étant intégrés dans les gabarits de fraisage (3), plusieurs bagues d'épaisseur (5) étant prévues, dont à chaque fois une peut être enfichée sur le corps de fraisage (4) de taille adaptée et dont l'épaisseur correspond à celle de la prothèse de rotule (99) à chaque fois associée au corps de fraisage.

2. Système d'instrument modulaire selon la revendication 1, **caractérisé en ce que** l'on prévoit sur les corps de fraisage (4), en plus de la bague d'épaisseur (5), une bague d'arrêt mobile (43).

3. Système d'instrument modulaire selon la revendication 2, **caractérisé en ce que** l'on prévoit un dispositif de serrage (44) sur la bague d'arrêt (43).

4. Système d'instrument modulaire selon la revendication 2 ou 3, **caractérisé en ce que** la bague d'arrêt (43) est réalisée de manière à coïncider avec la bague d'épaisseur (5).

5. Système d'instrument modulaire selon l'une quelconque des revendications précédentes, **caractérisé en ce que** l'instrument (1) présente une articulation (10) qui est réalisée en vue d'un guidage linéaire des organes (11, 12).

6. Système d'instrument modulaire selon l'une quelconque des revendications précédentes, **caractérisé en ce que** des inserts de gabarits de forage (6) sont prévus avec des tailles différentes.

7. Système d'instrument modulaire selon la revendication 6, **caractérisé en ce que** les inserts de gabarits de forage (6) sont pourvus d'un marquage angulaire (64).

8. Système d'instrument modulaire selon l'une quelconque des revendications précédentes, **caractérisé en ce que** l'on prévoit un dispositif de pressage (7) pouvant être appliqué sur le logement (18).

9. Système d'instrument modulaire selon l'une quelconque des revendications précédentes, **caractérisé en ce que** l'on prévoit un embout de forage (86) avec un foret (8) limité en profondeur par une butée.

10. Système d'instrument modulaire selon l'une quelconque des revendications précédentes, **caractérisé en ce que** l'on prévoit un module de gabarit de sciage (9) qui peut être appliqué sur le logement (18) de l'instrument de retenue (1).

11. Système d'instrument modulaire selon la revendication 10, **caractérisé en ce que** l'on prévoit des guides pour le module de gabarit de sciage (9) sur le module de gabarit de taille (2).

12. Système d'instrument modulaire selon la revendication 10 ou 11, **caractérisé en ce que** l'on prévoit un dispositif de mesure de profondeur sur le module de gabarit de sciage (9).

13. Système d'instrument modulaire selon la revendication 12, **caractérisé en ce que** le dispositif de mesure de profondeur présente un palpeur qui pénètre par le haut dans un remplissage formé par le gabarit de taille (2).

14. Système d'instrument modulaire selon la revendication 13, **caractérisé en ce que** le palpeur est réalisé sous forme de palpeur en étoile (96) avec une pluralité de corps de palpeur (98) disposés en faisceau, qui présentent chacun une taille différente.
